# EUROPEAN PATENT APPLICATION

(11) **EP 3 797 648 A1**
(43) Date of publication of application: **31.03.2021**
(21) Application number: 19808269.5
(22) Date of filing: 20.05.2019
(51) Int. Cl.: A47C 27/00, A47C 27/22

(54) **BEDDING**

(30) Priority: 22.05.2018 JP 2018098353
(71) Applicant: Delta Kogyo Co., Ltd., Aki-gun, Hiroshima 735-8501 (JP)
(72) Inventor: NISHIDA, Atsushi, Aki-gun, Hiroshima 735-8501 (JP); ASANO, Akihito, Aki-gun, Hiroshima 735-8501 (JP)
(74) Representative: Hoffmann Eitle
(86) International application number: PCT/JP2019/019908
(87) International publication number: WO 2019/225542

(57) **Abstract**

There is provided a bedding which allows comfortable sleep to be achieved. The bedding has a surface cushion member 1100 which has air permeability in the thickness direction and the plane direction and whose surface side is a contact surface with a human body, and thereunder, the bed internal environment control means which forms a stream of air in the surface cushion member 1100 is arranged. Accordingly, in the surface cushion member 1100, the air not only passes through the thickness direction but also quickly spreads in the plane direction, and an air layer in the surface cushion member 1100 is controlled to a predetermined temperature, humidity, or air flow, to generate the temperature gradient, the humidity gradient, or the air flow gradient between an air layer around the human body supported by the surface cushion member 1100 and the air layer in the surface cushion member 1100. As a result, a gentle movement of air is generated between the air layer around the human body and the air layer in the surface cushion member 1100, and the bed internal environment (temperature, humidity, or air flow) is gently controlled to a proper condition, which allows comfortable sleep to be achieved.

## Description

### Technical Field

The present invention relates to a bedding, and in particular relates to a bedding capable of controlling a bed internal environment.

### Background Art

Patent Document 1 discloses a bedding which sends a wind at a predetermined temperature to a mat having air permeability to control a bed internal temperature based on a temperature sensor disposed in the mat. Further, Patent Document 2 discloses a bedding apparatus which sends a wind at a predetermined temperature into a bag body supporting a person to control a bed internal temperature.

### Prior Art Document

### Patent Document

Patent Document 1: Japanese Patent Application Laid-open No. S58-25116
Patent Document 2: Japanese Patent Application Laid-open No. H3-41910

### Disclosure of the Invention

### Problems to Be Solved by the Invention

However, the techniques disclosed in Patent Documents 1 and 2 are the ones which use the mat and the bag body supporting a human body as, so to speak, a part of an airflow duct, and the winds blown off from the mat and the bag body directly act on the human body. Further, due to configurations in which these winds are sent in response to the bed internal temperatures, the human body is irregularly exposed to the winds while asleep, and moreover, their temperatures are not constant either, and if anything, the controls by using these winds are likely to lead to prevention of comfortable sleep.

The present invention was made in consideration of the above and has an object to provide a bedding which creates a temperature gradient, a humidity gradient, or an air flow gradient between an air layer around a human body on a surface cushion member and an air layer in the surface cushion member, thereby gently moving air between the periphery of the human body and the surface cushion member to control a bed internal environment, which allows comfortable sleep to be achieved.

### Means for Solving the Problem

To solve the above problems, a bedding of the present invention including:
a surface cushion member which has air permeability in a thickness direction and a plane direction and whose surface side is a contact surface with a human body;
a bed internal environment control means which is arranged under the surface cushion member, has a function of forming a stream of air in the surface cushion member, generates a temperature gradient, a humidity gradient, or an air flow gradient between an air layer around the human body on the contact surface and an air layer in the surface cushion member, and promotes a movement of air between the periphery of the human body and the surface cushion member to control a bed internal environment; and
a support layer which supports the surface cushion member and the bed internal environment control means and suppresses an influence of outside air on the surface cushion member side.

Preferably, the surface cushion member is formed by using a three-dimensional knitted fabric.

Preferably, the support layer is formed of a bead foam.

Preferably, the bed internal environment control means includes:
at least one ventilation mechanism which is arranged under the surface cushion member and has a body case whose interior is an air circulation space, a fan which is disposed in the body case and sucks air from the contact surface side with the human body of the surface cushion member into the body case, and an exhaust port which is provided to be open in a direction facing the contact surface with the human body of the surface cushion member at a position apart from the fan in the body case and discharges an air stream accompanying intake of the fan toward the contact surface with the human body;
a heater which warms the air stream;
a bed internal environment measuring sensor; and
a control unit which controls the ventilation mechanism and the heater based on a measured result of the bed internal environment measuring sensor.

Preferably, the heater is a flat heater arranged between the surface cushion member and at least one of the ventilation mechanisms.

Preferably, the ventilation mechanism further includes a swirling stream generation part which swirls the air stream to cause the air stream to be discharged as a swirling stream from the exhaust port.

Preferably, the swirling stream generation part has a cylindrical portion rising in a direction toward the exhaust port and generates the swirling stream by making the air stream swirl around the cylindrical portion.

Preferably, a biosignal measurement device which acquires a biosignal from a person supported by the surface cushion member is attached to the surface cushion member, and an analyzer which analyzes a biological state of the person from the biosignal is included to enable monitoring of the biological state of the person.

Preferably, the control unit has a function of controlling at least one of the ventilation mechanism and the fan based on the biological state of the person obtained from the analyzer.

### Effects of the Invention

The bedding of the present invention has the surface cushion member which has the air permeability in the thickness direction and the plane direction and whose surface side is the contact surface with the human body, and thereunder, the bed internal environment control means which forms the stream of air in the surface cushion member is arranged. Accordingly, in the surface cushion member, the air not only passes through the thickness direction but also quickly spreads in the plane direction, and the air layer in the surface cushion member is controlled to a predetermined temperature, humidity, or air flow, to generate the temperature gradient, the humidity gradient, or the air flow gradient between the air layer around the human body supported by the surface cushion member and the air layer in the surface cushion member. As a result, a gentle movement of air is generated between the air layer around the human body and the air layer in the surface cushion member, and the bed internal environment (temperature, humidity, or air flow) is gently controlled to a proper condition, which allows comfortable sleep to be achieved. Because using a three-dimensional fabric, in particular, the three-dimensional knitted fabric as the surface cushion member causes the air to flow more quickly in the plane direction through wide gaps between connecting yarns than a flow of air which directly acts on the human body through gaps between yarns of ground knitted fabrics, the above-described function is likely to be exhibited.

Further, the support layer supporting the surface cushion member and the bed internal environment control means has a function of suppressing the influence of outside air on the surface cushion member side. The support layer is preferably formed of the bead foam. This suppresses that the above-described movement of the air in the air layer in the surface cushion member and the air layer around the human body is prevented by the influence of outside air.

In addition, the bed internal environment control means is preferably configured to provide the ventilation mechanism which sucks the air from the contact surface side with the human body, and thereafter discharges the air toward the contact surface with the human body again. The suction of the air makes it possible to efficiently reduce humidity due to heat, sweat, and the like in the vicinity of the contact portion of the surface cushion member with the human body. Further, since a stream of the air discharged from the exhaust port comes into contact with the human body through the surface cushion member, the exhaust also enables the reduction in the heat and the humidity in the vicinity of the contact portion. In addition, having the configuration in which the air becomes the swirling stream to be discharged makes a flow mainly in a tangent direction more likely to be generated than in a normal direction with respect to a body surface, and allows the air layer near the body surface to be efficiently stirred, which enhances a reduction effect on the heat, the humidity, and the like, resulting in enabling improvement in comfort.

### Brief Description of Drawings

[FIG. 1] FIG. 1 is a perspective view illustrating a bedding according to one embodiment of the present invention.
[FIG. 2] FIG. 2 is a view illustrating an example of a disposition relationship between a ventilation mechanism and a heater.
[FIG. 3] FIG. 3(a) is a view schematically illustrating a partial cross section of the above-described embodiment.
[FIG. 4] FIG. 4 is a perspective view illustrating the ventilation mechanism used in the above-described embodiment.
[FIG. 5] FIG. 5 is a plan view of FIG. 4.
[FIG. 6] FIG. 6 is a side view of FIG. 4.
[FIG. 7] FIG. 7 is a rear view of FIG. 4.
[FIG. 8] FIG. 8 is a graph illustrating measured results of differences in temperatures depending on portions of a surface cushion member according to presence/absence of drive of fans in the ventilation mechanism.
[FIGs. 9] FIGs. 9(a), (b) are views illustrating an example of a biosignal measurement device.

### Description of Embodiment

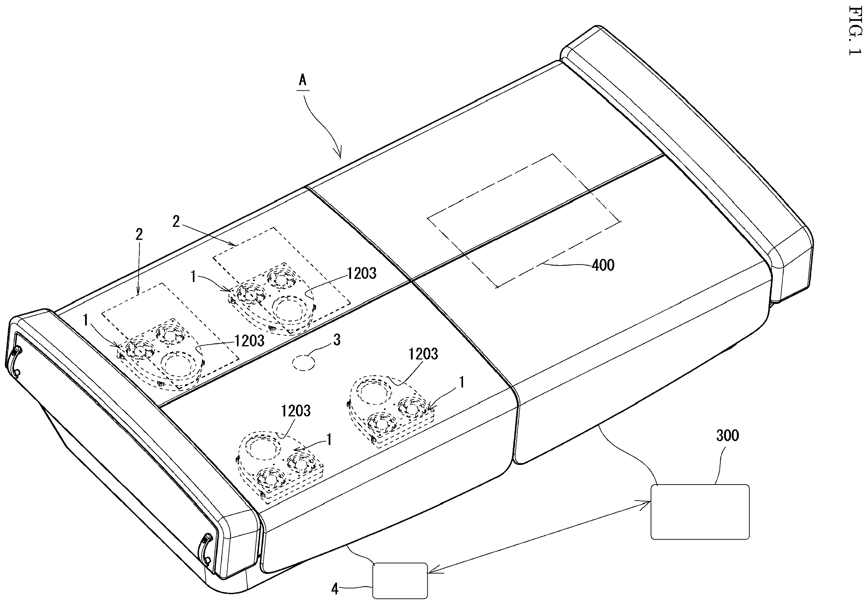

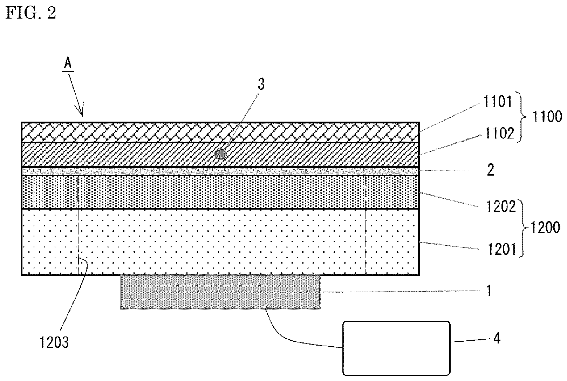

The present invention will be hereinafter described in more detail based on an embodiment illustrated in the drawings. FIG. 1 and FIG. 2 are views illustrating a bedding A according to one embodiment of the present invention. As illustrated in these views, the bedding A of this embodiment includes a surface cushion member 1100 and a support layer 1200, and between them, ventilation mechanisms 1 and heaters 2 composing a bed internal environment control means are arranged, and moreover, the bed internal environment control means has a bed internal environment measuring sensor 3 and a control unit 4.

The surface cushion member 1100 is disposed on a side coming into contact with a human body, and formed of a material having air permeability in a thickness direction and a plane direction. It is preferably a three-dimensional fabric having the air permeability and being excellent in a cushioning property, and more preferably a three-dimensional knitted fabric. Further, as the surface cushion layer 1100, the three-dimensional fabric such as the three-dimensional knitted fabric may be used in one piece, or as illustrated in FIG. 2, a stack of a plurality of the three-dimensional fabrics 1101, 1102 may be used. Moreover, a stack in combination with an air-permeable two-dimensional fabric, a different kind of three-dimensional fabric, or the like may be used. Note that the three-dimensional knitted fabric mentioned here is formed three-dimensionally by connecting yarns reciprocating between two layers of ground knitted fabrics at a predetermined interval, has a cushioning property large enough to support a person's load even if it is thin, for example, even if its thickness is about 3 to 20 mm, owing to the rigidity of the ground knitted fabrics, the elasticity of the connecting yarns, and so on, and can alleviate a feeling of something foreign of the ventilation mechanism 1 and the like arranged thereunder. Further, it is possible to exhibit high air permeability both in the thickness direction and in the plane direction through gaps between yarns of ground knitted fabrics and gaps between connecting yarns. However, because the gap between the connecting yarns is larger than the gap between the yarns between the ground knitted fabrics, a spread of air in the plane direction is smoother.

The support layer 1200 is disposed in a lower side of the surface cushion member 1100, and supports the bed internal environment control means such as the ventilation mechanism 1 and the surface cushion member 1100. The support layer 1200 has a function of suppressing an influence of outside air on the surface cushion member 1100 side, preferably shutting off the outside air. Preferably, a bead foam 1201 excellent in a heat-insulating property and a heat-retaining property is used. The support layer 1200 only needs to have such a function, and can also be composed of only the bead foam 1201, but as described in this embodiment, in order to make up for a feeling of stroke of a person supported on the surface cushion member 1100, on the bead foam 1201, for example, an open-cell urethane foam material 1202 with a predetermined thickness can also be arranged.

The ventilation mechanism 1 is arranged in a concave portion for arrangement 1203 formed in an appropriate position of the support layer 1200. The formation number of concave portions for arrangement 1203 is not limited, and is determined in consideration of a size of the ventilation mechanism 1, or the like, and for example, the ventilation mechanism 1 can be formed in one piece near the middle of the bedding A, or as illustrated in FIG. 1, can be formed in plurality. The concave portion for arrangement 1203 may pass through from a front surface side of the urethane foam material 1202 to a rear surface side of the bead foam 1201 (see FIG. 2), or may be dented halfway in the thickness direction of the bead foam 1201.

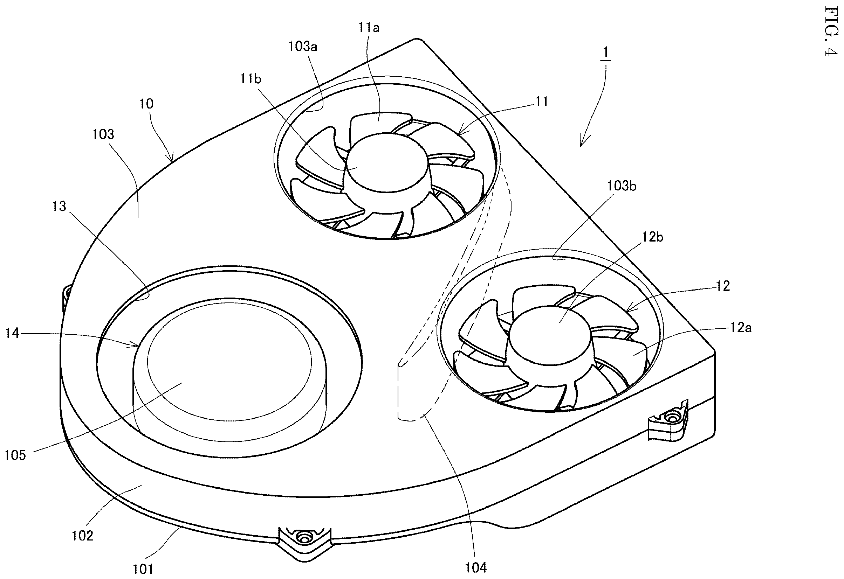

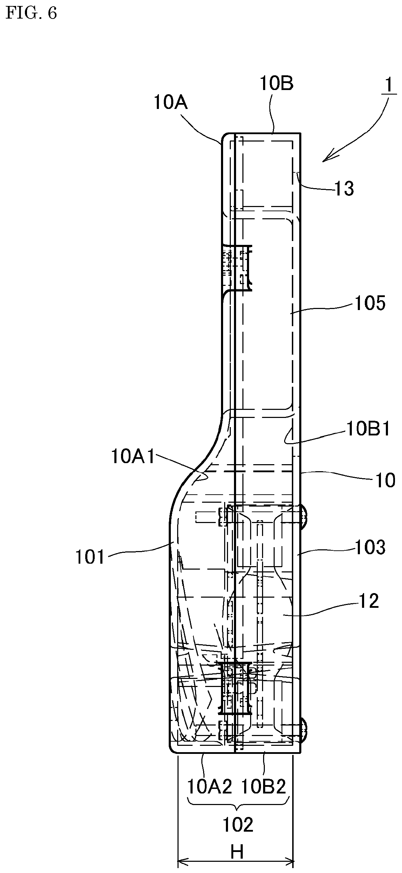

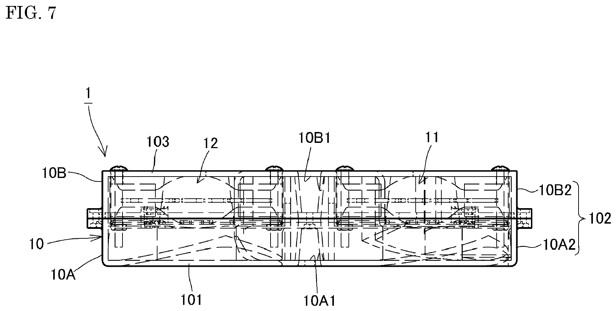

The ventilation mechanism 1 has a body case 10, two fans 11, 12, an exhaust port 13, and a swirling stream generation part 14 as illustrated in FIG. 4 to FIG. 7. The body case 10 has two concave plate-shaped members 10A, 10B which include concave portions 10A1, 10B1 inside, the concave plate-shaped members 10A, 10B are joined with their open surfaces facing each other, and an interior space formed by the concave portions 10A1, 10B1 is an air circulation space. The body case 10 is composed of the joined two concave plate-shaped members 10A, 10B, a wall portion corresponding to a bottom surface of the concave portion 10A1 of the concave plate-shaped member 10A is a bottom wall portion 101 of the entire body case 10, and a wall portion corresponding to a bottom surface of the concave portion 10B1 of the other concave plate-shaped member 10B is an upper wall portion 103, of the entire body case 10, located on a contact surface side with a person. Further, when the two concave plate-shaped members 10A, 10B are joined with their open surfaces facing each other, their peripheral walls 10A2, 10B2 form a peripheral wall portion 102 of the entire body case 10. In the upper wall portion 103, intake ports 103a, 103b are opened at positions corresponding to arrangement positions of the two fans 11, 12.

Having the structure in which the fans 11, 12, the exhaust port 13, and the swirling stream generation part 14 are provided in the body case 10, the ventilation mechanism 1 of this embodiment is formed as a unit. Therefore, the ventilation mechanism 1 can be simply used by being disposed as a unit in the above-described concave portion for arrangement 1203.

The two fans 11, 12 have a plurality of rotary blades 11a, 12a, and when they rotate, air is sucked from a front surface side to a rear surface side of the rotary blades 11a, 12a. The two fans 11, 12 are arranged side by side in the width direction on one length-direction end side in the body case 10 in a direction in which the rotary shafts 11b, 12b supporting the rotary blades 11a, 12a are substantially perpendicular to the bottom wall portion 101.

The exhaust port 13 is provided at a position apart from the fans 11, 12 in a plan view so as to be open in an upper direction being a direction facing the contact surface with a human body in the surface cushion member 1100. Accordingly, in this embodiment, the exhaust port 13 is formed to be open in the upper wall portion 103 of the body case 10 together with the intake ports 103a, 103b corresponding to the fans 11, 12. The fans 11, 12 and the intake ports 103a, 103b are provided on the length-direction one end side of the body case 10 as described above, and the exhaust port 13 is provided at a position a predetermined distance apart therefrom toward the length-direction other end side.

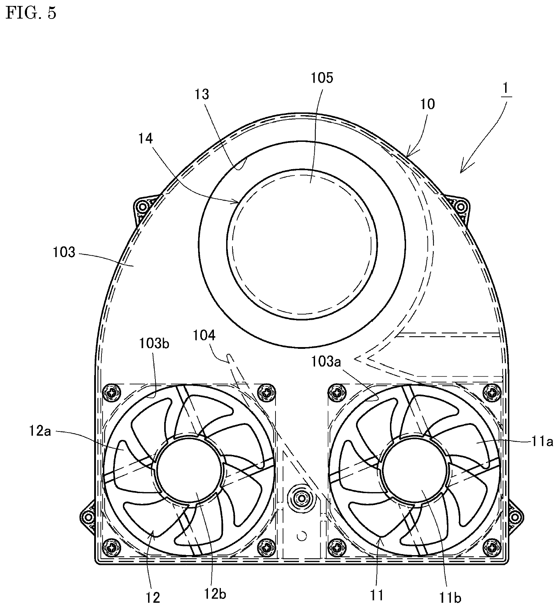

Here, in the body case 10, a guide wall 104 is provided to make the air sucked by the fans 11, 12 easily flow toward the exhaust port 13 in the air circulation space surrounded by the bottom wall portion 101, the peripheral wall portion 102, and the upper wall portion 103. As illustrated in FIG. 5, in a plan view, the guide wall 104 is substantially arc-shaped and extends to project toward the exhaust port 13. Accordingly, the air sucked by the fan 11 tries to diffuse toward the peripheral wall portion 102 but is restricted by the guide wall 104 to flow in a swirling manner. Further, as illustrated in FIG. 5, in the peripheral wall portion 102, its portion close to the exhaust port 13 is formed in a substantially semicircular shape in a plan view. Accordingly, the air sucked by the other fan 12 also diffuses toward the peripheral wall portion 102 but is restricted by the peripheral wall portion 102 to flow in a swirling manner.

The opening of the exhaust port 13 is substantially circular, and a cylindrical portion 105 substantially concentric with the exhaust port 13 and smaller in outside diameter than the exhaust port 13 in a plan view is provided upright on the bottom wall portion 101. The air sucked by the fans 11, 12 is restricted by the guide wall 104 and the peripheral wall portion 102 to be the swirling stream as described above, and owing to such a cylindrical portion 105, in a lower part of the exhaust port 13, the stream of the air sucked by the fans 11, 12 becomes a swirling stream swirling around the cylindrical portion 105, and the swirling stream is discharged from the exhaust port 13. Therefore, when the air is discharged as the swirling stream from the exhaust port 13, around the exhaust port 13 and in the vicinity of the contact surface with the human body in the surface cushion member 1100, a flow mainly in a tangent direction is more easily generated than in a normal direction to the human body, and an air layer near the body surface around the human body can be efficiently stirred, which enhances a reduction effect on heat, humidity, and the like, resulting in enabling improvement in comfort. In this embodiment, the swirling stream generation part 14 is composed of the combination of the cylindrical portion 105 with the peripheral wall portion 102 and the guide wall 104 of the body case 10.

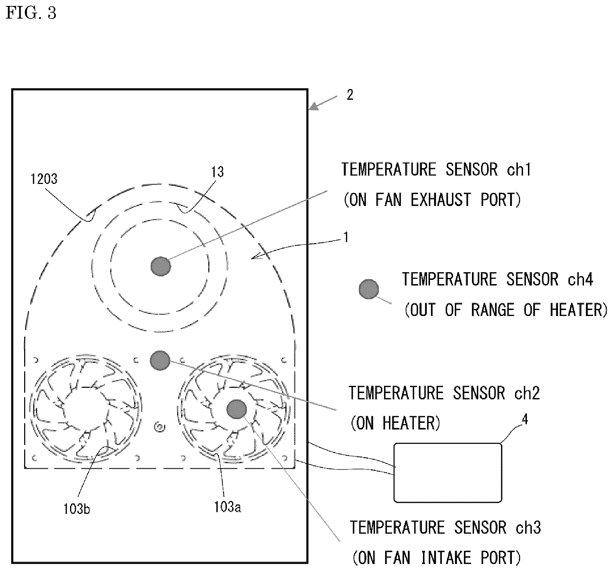

The heater 2 is composed of a thin sheet-shaped one (flat heater), and covers the concave portion for arrangement 1203 to be stacked on a surface of the urethane foam material 1202 of the support layer 1200 between the surface cushion member 1100 and the ventilation mechanism 1 (see FIG. 2, FIG. 3). This prevents close contact with the intake ports 103a, 103b and the exhaust port 13 of the ventilation mechanism 1, to create a gap. Further, a gap is provided so that a part of a peripheral edge of the concave portion for arrangement 1203 is not occupied by the heater 2 either. This makes an air stream come in contact with the heater 2 without preventing the intake by using the fans 11, 12 and the exhaust. In order to make the intake by using the fans 11, 12 and the exhaust smooth, as the flat heater composing the heater 2, one having through holes in a range corresponding to positions of the intake ports 103a, 103b and the exhaust port 13, a net-shaped one in which band-shaped ones are disposed at predetermined intervals, or the like can be used.

The heater 2 may be disposed corresponding to all the arranged ventilation mechanisms 1, or may be disposed corresponding to only a part of them. Alternatively, it can also be provided in a position slightly deviating from an arrangement position of the ventilation mechanism 1. This is because the surface cushion member 1100 has the air permeability in the thickness direction and the plane direction and because the air sucked from the intake ports 103a, 103b by drive of the fans 11, 12 of the ventilation mechanism 1 is discharged from the exhaust port 13 to thereafter move quickly in the plane direction through the gaps particularly between the connecting yarns of the surface cushion member 1100. In FIG. 1, the two heaters 2 are arranged corresponding to the two ventilation mechanisms 1, but this is strictly an example, and for example, it is possible to dispose the heater 2 corresponding to the ventilation mechanism 1 located near a person's feet, or, conversely, to dispose it corresponding to the ventilation mechanism 1 located near a person's waist.

The bed internal environment measuring sensor 3 is arranged in the vicinity of the surface cushion member 1100. Because there is a space formed by a comforter (not illustrated) and a mattress (corresponding to the surface cushion member 1100) in the bedding, the bed internal environment measuring sensor 3 is provided on a surface of the surface cushion member 1100 or at a position near the surface within a thickness of the surface cushion member 1100. As the bed internal environment measuring sensor 3, there can be cited a temperature sensor, a humidity sensor, an air flow sensor, or the like.

The control unit 4 performs the control (ON•OFF, rotational speed, rotation time) of the fans 11, 12 of the ventilation mechanism 1 or the control (ON•OFF, temperature, warming time) of the heater 2 based on a temperature, a humidity, or an air flow measured by the bed internal environment measuring sensor 3. In general, because a proper bed internal temperature is said to be 33°C±1°C, and a proper relative humidity in bed is said to be 50%±5%, the control unit 4 controls measured values by the bed internal environment measuring sensor 3 to approximate the above values.

According to this embodiment, when the bed internal environment measuring sensor 3 judges the bed internal temperature to be higher than the above proper temperature, for example, the control unit 4 drives the fans 11, 12 of the ventilation mechanism 1 to be rotated. Driving the fans 11, 12 causes the air to be sucked from the intake ports 103a, 103b into the body case 10 formed by including the bottom wall portion 101, the peripheral wall portion 102, and the upper wall portion 103. The sucked air circulates in the air circulation space surrounded by the bottom wall portion 101, the peripheral wall portion 102, and the upper wall portion 103 in the body case 10, and swirls around the cylindrical portion 105 composing the swirling stream generation part 14 to be discharged from the exhaust port 13. The air is sucked from the contact surface side with the human body in the surface cushion member 1100, thereby quickly reducing the heat and the sweat. Further, a stream of the air discharged from the exhaust port 13 quickly spreads in the surface cushion member 1100, to generate a temperature gradient, a humidity gradient, or an air flow gradient between an air layer around the human body and the air layer in the surface cushion member 1100 as well. That promotes a stream of the air between the air layer around the human body and the air layer in the surface cushion member 1100, which quickly lowers the bed internal temperature.

On the other hand, when the bed internal environment measuring sensor 3 judges the bed internal temperature to be lower than the above proper temperature, the control unit 4 turns the heater 2 ON to make the heater generate heat, and drives the fans 11, 12 of the ventilation mechanism 1 to be rotated. This generates such a stream of the air that the air is sucked into the body case 10 through the intake ports 103a, 103b to be thereafter discharged from the exhaust port 13, during which the air is warmed by the heater 2. The warmed air not only passes through above the surface cushion member 1100 but also quickly spreads across in the plane direction in the surface cushion member 1100. That generates the temperature gradient, the humidity gradient, or the air flow gradient between the air layer around the human body located on the contact surface side with the surface cushion member 1100 and the air layer in the surface cushion member 1100. Therefore, in a wide range of the surface cushion member 1100, a gentle stream of air between the air layer in the surface cushion member 1100 and the air layer around the human body is generated, resulting in a rise in the bed internal temperature.

Thus, according to this embodiment, using the temperature gradient, the humidity gradient, or the air flow gradient between the human body side separated by the surface cushion member 1100 and the interior of the surface cushion member 1100 makes it possible to gently guide the air layer around the human body to a proper temperature or humidity, and to properly maintain the bed internal environment without preventing comfortable sleep.

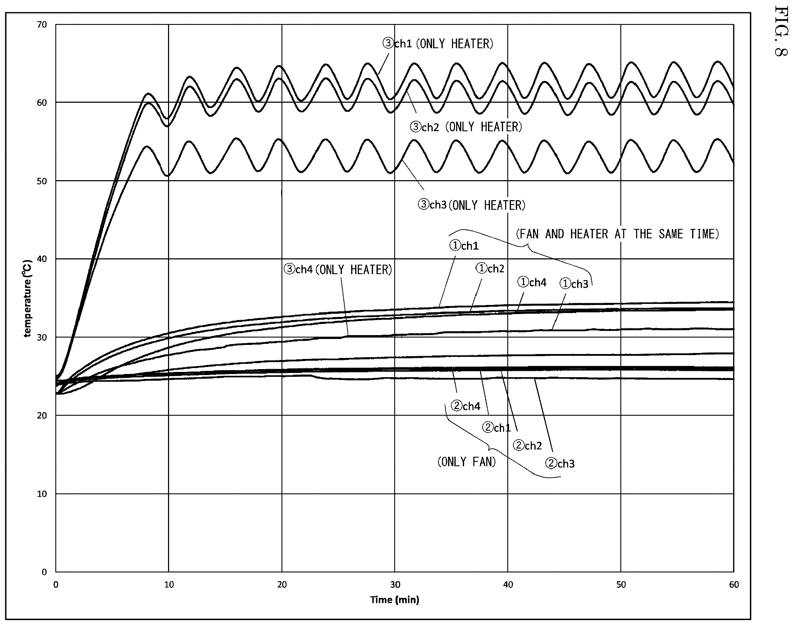

Here, with the temperature sensors set at positions of chl to ch4 illustrated in FIG. 3, regarding a case of operating only the fans 11, 12 of the ventilation mechanism 1, a case of operating only the heater 2, and a case of operating the fans 11, 12 and the heater 2 at the same time, temperature changes were measured. Note that the measurement was carried out without making a person lie on the surface cushion member 1100 and without disposing a comforter thereon. FIG. 8 illustrates results.

As is apparent from FIG. 8, in the case of operating only the fans 11, 12, between the temperature sensor ch1 placed near the exhaust port 13, the temperature sensor ch2 placed near the intake port 103a, the temperature sensor ch3 placed at a substantially intermediate position between them, and the temperature sensor ch4 placed at a position outward apart from both an outer periphery of the ventilation mechanism 1 and an outer periphery of the heater 2, there was little difference in the measurement.

On the other hand, in the case of operating only the heater 2, values in the temperature sensor ch1 placed near the exhaust port 13 and the the temperature sensor ch3 placed at the substantially intermediate position were almost the same, and a value in the temperature sensor ch2 placed near the intake port 103a was about 10°C lower than those in the temperature sensors ch1, ch3, and as for the temperature sensor ch4 placed at the position outward apart from the heater 2, a value was further 25°C or more lower than that in the temperature sensor ch2. In contrast to this, in the case of operating the fans 11, 12 in addition to the heater 2 together, values in ch1 to ch4 fell in almost the same range (a temperature difference was about 5°C even at the maximum). This indicates that the stream of the air spreads across in the plane direction in the surface cushion member 1100 by using the fans 11, 12, and also in a case of warming, it is found preferable that only the heater 2 is not operated but it is operated simultaneously with the fans 11, 12.

Incidentally, in the above-described embodiment, the two fans 11, 12 are used and the exhaust port 13 is set at one section, but as long as the ventilation mechanism 1 has a structure having an intake function and an exhaust function and being capable of generating the swirling stream at the time when the air is discharged, the displacement number of fans may be one or may be three or more. The formation number of exhaust ports may also be plural.

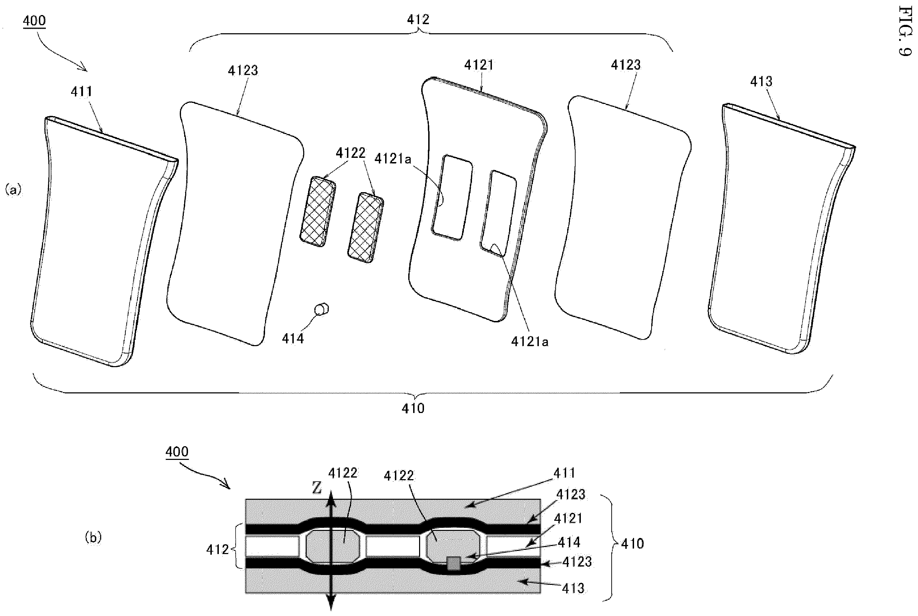

FIGs. 9(a), (b) are views illustrating a biosignal measurement device 400 which acquires a biosignal from a person supported by the surface cushion member 1100. The biosignal measurement device 400 is disposed under the surface cushion member 1100 and in a position corresponding to a person's back different from positions of the above-described ventilation mechanism 1 and the heater 2 (see FIG. 1). The biosignal measurement device 400 has a biosignal detection unit 410 in addition to not-illustrated electrical wiring or the like.

The biosignal detection unit 410 has a three-layer structure composed of a stack of a first layer member 411, a second layer member 412, and a third layer member 413 which each have a substantially rectangular shape having predetermined width and length. The first layer member 411 is formed of a three-dimensional knitted fabric or the like, and it is used while being placed on a side toward the human body whose biosignal is to be detected, and the biosignal is first propagated to the first layer member 411 through the dorsal body surface of the human body . The second layer member 412 functions as a resonance layer which emphasizes a weak dorsal body surface pulse wave propagated from the first layer member 411, by a resonance phenomenon or a beat phenomenon, and includes a base member 4121 formed of a bead foam or the like, three-dimensional knitted fabrics 4122 functioning as natural oscillators, and films 4123 generating membrane vibration. In the base member 4121, two placement holes 4121a, 4121a are formed at symmetrical positions sandwiching its center, and the three-dimensional knitted fabrics 4122, 4122 functioning as the natural oscillators are placed in the placement holes 4121a, 4121a. The films 4123, 4123 are stacked on surfaces of the second layer member 412 to cover exposed surfaces of the three-dimensional knitted fabrics 4122, 4122 functioning as the natural oscillators. Then, between one of the three-dimensional knitted fabrics 4122 and the film 4123, a microphone sensor 414 which detects vibration (sound) ascribable to the dorsal body surface pulse wave is disposed. The third layer member 413 is stacked on a side opposite to the first layer member 411 with the second layer member 412 therebetween and reduces an external vibration input. The third layer member 413 preferably has a function of damping external vibration with high frequencies of over 100 Hz. The third layer member 413 is preferably formed of a three-dimensional knitted fabric similarly to the first layer member 411 in order to have such a filtering function.

A signal from the biosignal measurement device 400 is sent to an analyzer 300 (see FIG. 1), which analyzes a biological state such as, for example, sleepiness, fatigue degree, heart rate, respiratory rate, body temperature, blood pressure, and sweat volume. By displaying the analysis result in, for example, a monitor (not illustrated), it is possible to find out a biological state of a person lying on the bedding A.

Further, it is also possible to have a configuration of not only simply performing monitoring but also outputting a signal to operate the ventilation mechanism 1 or the heater 2 from the control unit 4 based on the analysis result. For example, based on that the blood pressure rises and falls compared with a predetermined set value, the ventilation mechanism 1 or the heater 2 can be driven. Alternatively, in a case where the sweat volume is equal to or more than a predetermined volume, the ventilation mechanism 1 can also be operated. Such control also makes it possible to properly guide the bed internal environment and promote comfortable sleep.

### Explanation of Reference Signs

- A: bedding
- 1100: surface cushion member
- 1200: support layer
- 1: ventilation mechanism
- 10: body case
- 101: bottom wall portion
- 102: peripheral wall portion
- 103: upper wall portion
- 104: guide wall
- 105: cylindrical portion
- 11, 12: fan
- 13: exhaust port
- 14: swirling stream generation part
- 2: heater
- 3: bed internal environment measuring sensor
- 4: control unit
- 300: analyzer
- 400: biosignal measurement device

## Claims

1. A bedding comprising:
a surface cushion member which has air permeability in a thickness direction and a plane direction and whose surface side is a contact surface with a human body;
a bed internal environment control means which is arranged under the surface cushion member, has a function of forming a stream of air in the surface cushion member, generates a temperature gradient, a humidity gradient, or an air flow gradient between an air layer around the human body on the contact surface and an air layer in the surface cushion member, and promotes a movement of air between the periphery of the human body and the surface cushion member to control a bed internal environment; and
a support layer which supports the surface cushion member and the bed internal environment control means and suppresses an influence of outside air on the surface cushion member side.

2. The bedding according to claim 1, wherein the surface cushion member is formed by using a three-dimensional knitted fabric.

3. The bedding according to claim 1 or 2, wherein the support layer is formed of a bead foam.

4. The bedding according to any one of claims 1 to 3, wherein
the bed internal environment control means includes:
at least one ventilation mechanism which is arranged under the surface cushion member and has a body case whose interior is an air circulation space, a fan which is disposed in the body case and sucks air from the contact surface side with the human body of the surface cushion member into the body case, and an exhaust port which is provided to be open in a direction facing the contact surface with the human body of the surface cushion member at a position apart from the fan in the body case and discharges an air stream accompanying intake of the fan toward the contact surface with the human body;
a heater which warms the air stream;
a bed internal environment measuring sensor; and
a control unit which controls the ventilation mechanism and the heater based on a measured result of the bed internal environment measuring sensor.

5. The bedding according to claim 4, wherein the heater is a flat heater arranged between the surface cushion member and at least one of the ventilation mechanisms.

6. The bedding according to claim 4 or 5, wherein the ventilation mechanism further includes a swirling stream generation part which swirls the air stream to cause the air stream to be discharged as a swirling stream from the exhaust port.

7. The bedding according to claim 6, wherein the swirling stream generation part has a cylindrical portion rising in a direction toward the exhaust port and generates the swirling stream by making the air stream swirl around the cylindrical portion.

8. The bedding according to any one of claims 1 to 7, wherein a biosignal measurement device which acquires a biosignal from a person supported by the surface cushion member is attached to the surface cushion member, and an analyzer which analyzes a biological state of the person from the biosignal is included to enable monitoring of the biological state of the person.

9. The bedding according to claim 8, wherein the control unit has a function of controlling at least one of the ventilation mechanism and the fan based on the biological state of the person obtained from the analyzer.
